# FASCICULE DE BREVET EUROPEEN

(11) **EP 1 014 899 B1**
(45) Date de publication et mention de la délivrance du brevet: **18.08.2004**
(21) Numéro de dépôt: 97919092.3
(22) Date de dépôt: 12.09.1997
(51) Int. Cl.: A61F 2/44

(54) **CAGE D'OSTEOSYNTHESE EXPANSIVE**
EXPANDIERBARER OSTEOSYNTHESEKÄFIG
EXPANSIVE OSTEOSYNTHESIS SHEATH

(30) Priorité: 13.09.1996 FR 9611452
(43) Date de publication de la demande: 05.07.2000
(73) Titulaire: Cage Concepts LLC, Laguna Hills, CA 92653 (US); Attali, David, 84000 Avignon (FR); Attia, David, 26200 Montélimar (FR); Chauvin, Jean Luc, 84100 Uchaux (FR)
(72) Inventeur: CHAUVIN, Jean-Luc, F-84100 Uchaux (FR); ATTALI, David, F-84000 Avignon (FR); ATTIA, David, F-26200 Montélimar (FR)
(74) Mandataire: Domange, Maxime
(86) Numéro de dépôt international: PCT/FR1997/001617
(87) Numéro de publication internationale: WO 1998/010722

(56) Documents cités:
- EP-A- 0 637 440
- EP-A- 0 664 994
- DE-C- 4 416 605

## Description

La présente invention a pour objet une cage d'ostéosynthèse expansive.

Le secteur technique de l'invention est le domaine des implants ou prothèses osseuses implantables et les procédés chirurgicaux pour leur mise en oeuvre.

L'application principale de l'invention est la réalisation d'implants destinés à être glissés ou insérés par voie postérieure entre les plateaux vertébraux de deux vertèbres consécutives pour maintenir une distance donnée entre eux et rétablir la stabilité de la colonne vertébrale, suite par exemple à une défaillance de l'articulation correspondante, en rendant alors solidaires les deux vertèbres.

On connaît en effet à ce jour plusieurs techniques pour rétablir ainsi la lordose lombaire « normale », en implantant soit un greffon qui réalise dans le temps une fusion entre chaque vertèbre, soit un matériel prothésique immobilisant immédiatement celles-ci, tout en permettant de réaliser aussi dans le temps une fusion entre les vertèbres.

Dans cette dernière technique, on utilise principalement des implants appelés également « cages », dont certains sont creux, rigides et monoblocs, avec des lumières de communication internes-externes, pour y loger un greffon osseux qui assure, à travers lesdites lumières, la fusion ultérieure dudit greffon avec les vertèbres adjacentes de part et d'autre : on peut citer dans ce domaine la demande de brevet WO 96/08205 publiée le 21 mars 1996 sur une « Cage de fusion intervertébrale de forme conique » et la demande EP 637 440 publiée le 8 février 1995 sur un « Implant intersomatique pour colonne vertébrale » ; ces types de cages sont cependant de dimensions externes données et fixes, alors que les distances entre deux vertèbres ne sont pas constantes ; de plus, l'inclinaison des plateaux intervertébraux auxquels on veut redonner une position angulaire donnée, ne permet pas d'utiliser des cases rigides par voie postérieure ; elles ne peuvent être introduites que par voie antérieure.

Aussi, il a été développé d'autres types de cages comportant deux branches sensiblement parallèles et reliées à un corps rigide à travers lequel on peut tourner, un système de vis sans fin qui rapproche alors un coin, vissé sur ladite vis et placé initialement du côté des extrémités distales des branches, vers leur corps de liaison, ce qui écarte les deux branches angulairement : on peut alors introduire une telle cage de forme initiale aplatie entre les vertèbres, puis en vissant l'axe de manoeuvre du coin, on règle et on impose un angle voulu entre les branches depuis un accès postérieur.

De telles cages ou implants sont décrits à titre d'exemple dans la demande de brevet EP 664 994 publiée le 2 août 1995, intitulée : « Cage intersomatique vertébrale », ou dans la demande EP 2.719.763 publiée le 17 novembre 1995 et intitulée : « Implant vertébral ».

Cependant, de tels dispositifs plus mécaniques que les cages creuses et rigides, sont donc plus complexes, offrent un volume intérieur plus faible pour un greffon de fusion et de par leur forme aplatie et non cylindrique de révolution, certes pour mieux assurer un angle d'appui donné entre les vertèbres, nécessitent la préparation d'un passage de même section rectangulaire pour les insérer, ce qui complique la mise en oeuvre.

Le problème posé est donc de pouvoir disposer d'implants ou cages permettant à la fois d'allier la géométrie d'une cage rigide classique, d'une part pour faciliter leur implantation et d'autre part pour offrir un grand volume intérieur, et la possibilité d'augmenter jusqu'à une valeur donnée le diamètre de l'extrémité distale de la cage par rapport à l'extrémité située du côté de son point d'insertion chirurgicale, une fois mis en place, et correspond à la face postérieure des vertèbres, tout en ayant le moins d'éléments mécaniques.

Une solution au problème posé est un implant d'ostéosynthèse expansif comprenant des branches reliées à l'une de leurs extrémités à un culot percé d'un orifice, tel que lesdites branches et le culot constituent une cage creuse dont, en position de « repos », la forme générale extérieure est un cylindre ou quasi cylindre dont la génératrice qui l'engendre en tournant autour de son axe de symétrie est, soit une droite, soit présente en sa partie médiane une portion de courbe telle qu'un arc de cercle convexe de grand rayon et dont la directrice sur laquelle s'appuie la génératrice, qui est aussi la section transversale du cylindre, est quasi circulaire : on obtient ainsi, un cylindre, soit régulier de révolution, soit que l'on qualifiera dans la présente description d'« ovoïde » ou d'« ovale » et que l'on désignera sous le terme cylindre ou quasi cylindre ; une partie au moins du volume intérieur de la cage vers les extrémités distales des branches, est en forme de tronc de cône quasi de révolution dont la plus grande base est du côté dudit culot, lequel implant comprend au moins trois branches et au moins une cale dont les dimensions sont compatibles avec celles de la grande base du tronc de cône dans ledit volume intérieur en position repos, et éventuellement apte à passer par ledit orifice.

En position « active », ladite cale écarte lesdites branches, ledit volume intérieur tend vers un cylindre de révolution, et la forme extérieure de la cage vers un quasi tronc de cône, et une fois la cale disposée à la position voulue, aucune pièce interne ne subsiste dans l'espace délimité par lesdites branches, la cale et l'orifice.

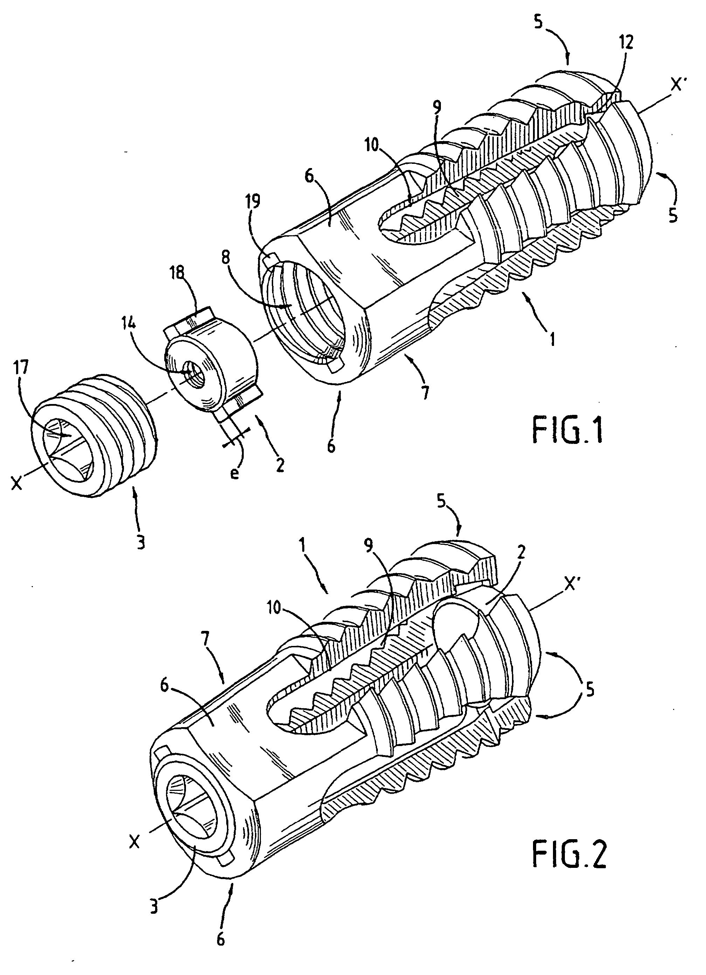

Les définitions des positions au « repos » et « active » sont représentées à titre d'exemple respectivement dans les figures 1, 3A, 4A, 5A, 9, 10, 11 et 2, 3B, 3C, 4B et 5B : la position au « repos » est en effet la position de l'implant avant sa mise en place et durant celle-ci, soit sans la cale positionnée entre ses branches à l'intérieur de la cage qui a donc un volume extérieur cylindrique de section constante ; alors que la position dite « active » est la position finale de l'implant entre par exemple deux vertèbres, les branches étant écartées par la cale placée en sa position de réglage final, la forme extérieure générale de la cage étant alors quasi tronconique, s'évasant depuis le culot correspondant à l'extrémité de l'implant situé du côté de son point d'insertion chirurgicale vers l'extrémité distale placée le plus à l'intérieur entre les vertèbres.

Pour assurer un meilleur ancrage dans l'os et afin d'éviter ultérieurement toute migration de la cage, de préférence, la surface extérieure desdites branches est soit moletée, soit rainurée, soit filetée suivant un profil de filet à arêtes saillantes, ou autres.

Pour réduire également les risques de rotation après implantation et donc de déplacement de la cage tout en augmentant la surface de contact avec les plateaux vertébraux, le culot au moins de l'implant si ce n'est même les côtés des branches, comportent au moins deux méplats parallèles ou non, et chacun disposé symétriquement par rapport à l'axe de l'implant entre deux branches successives ; ces méplats, complétés éventuellement par la forme ovoïde générale de la cage, permettent un meilleur maintien après mise en expansion en réduisant les risques de rotation de l'implant. De plus, ladite forme ovoïde peut permettre, mieux que si la génératrice extérieure de la forme du cylindre de base de la cage au repos est une droite, de revenir après expansion à un profil extérieur conique sans courbure et donc d'assurer un appui mieux réparti sur le corps vertébral et une bonne prise de greffe osseuse.

Pour rigidifier la cage à son extrémité postérieure et cela d'autant plus si l'on dispose d'un grand orifice dans le culot, ce qui permet d'assurer un remplissage plus aisé de la cage par de la matière osseuse, après mise en place et expansion de la cage, l'orifice dudit culot est apte à recevoir un bouchon de fermeture du volume intérieur de la cage : celui-ci peut se visser par exemple dans ledit orifice, alors également fileté ; dans tous les cas, ce bouchon empêche la matière osseuse de ressortir et suivant sa matière, il rigidifie également la cage.

Différents mode de réalisation particuliers sont décrits ci-après dans les figures ci-jointes. Le résultat est de nouveaux implants d'ostéosynthèse expansifs répondant au problème posé, en particulier pour l'application principale définie précédemment.

En effet, la présence d'au moins quatre branches et éventuellement de quatre à huit, permet d'avoir une expansion bilatérale, d'où un meilleur blocage sur les parois des deux plateaux vertébraux face à face, et l'absence de tige ou pièce de liaison, entre la cale maintenant ladite expansion et l'orifice ou le bouchon du culot d'extrémité, assure un grand volume intérieur permettant de recevoir une quantité importante de matière osseuse, ce qui améliore la consolidation et la jonction par fusion en particulier entre les vertèbres que l'on veut maintenir ; ledit remplissage étant d'autant plus aisé que l'on dispose d'un grand orifice à l'extrémité postérieure de la cage dans ledit culot.

De plus, ledit orifice permet de cureter les plateaux vertébraux au travers des rainures situées sur les faces inférieure et supérieure de la cage entre chaque branche.

De plus, le système d'expansion ainsi défini est très simple puisqu'il ne comporte que deux pièces qui sont d'une part la cage d'extrémité à laquelle on adjoint un bouchon et éventuellement une cale intermédiaire comme indiqué ci-après, mais sans organe de liaison restant après la mise en place, puisque toute tige ou axe permettant de positionner lesdites cales et ledit bouchon est ensuite enlevée.

La présente invention apporte donc de nombreux avantages par rapport aux implants et cages existantes, tels que ceux déjà évoqués que l'on pourrait compléter par d'autres mais qui en montrent déjà suffisamment pour en prouver la nouveauté et l'intérêt.

La description et les figures ci-après représentent deux exemples de réalisation de l'invention avec quatre branches, mais n'ont aucun caractère limitatif : d'autres réalisations sont possibles, dans le cadre de la portée et de l'étendue de cette invention, telles que par exemple avec trois branches ou plus de quatre.
La figure 1 est une vue perspective d'un exemple d'implant en position de « repos » et avec ses différentes pièces alignées suivant le même axe XX' de mise en place.
La figure 2 est une vue perspective de l'implant de la figure 1, en position « active », avec les trois pièces précédentes assemblées.
Les figures 3A, 4A et 5A sont respectivement une vue en coupe, et deux vues axiales latérales, l'une suivant la face antérieure et l'autre suivant la face postérieure d'une cage d'un autre mode de réalisation d'un implant suivant l'invention en position de « repos ».
Les figures 3B, 4B et 5B représentent suivant les mêmes vues, la même cage avec ses éléments complémentaires assemblés que celle représentée sur les figures 3A, 4A et 5A, mais en position « active ».
La figure 3C est une vue latérale perpendiculaire à l'axe de l'implant du mode de réalisation des figures 3, 4 et 5 en position « active ».
Les figures 6, 7 et 8 sont des figures représentant des détails de réalisation de la cage d'implant suivant l'invention.
Les figures 9, 10 et 11 sont respectivement une vue en coupe partielle latérale, et des vues axiales suivant la face antérieure et la face postérieure du mode de réalisation de l'implant représenté en perspective sur les figures 1 et 2.
Les figures 12, 13 et 14 représentent une vue de face et une vue en coupe respectivement d'une cale d'extrémité non vissée pour les modes de réalisation des figures 1, 2, 9, 10 et 11, une cale d'extrémité et/ou intermédiaire vissée d'un mode de réalisation suivant les figures 3, 4 et 5 et d'un bouchon de fermeture de l'un quelconque des modes de réalisation suivant l'invention.

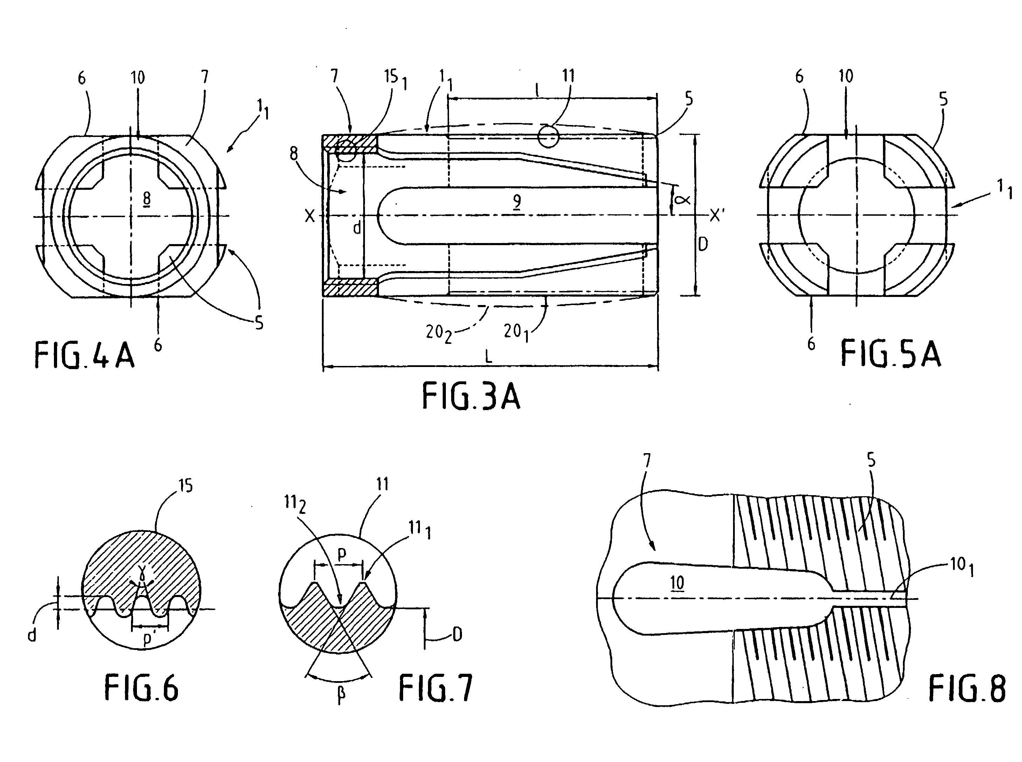

Quel que soit le mode de réalisation, l'implant d'ostéosynthèse expansif comprend d'une manière connue des branches 5 reliées à l'une de leurs extrémités à un culot 7 percé d'un orifice 8 ; suivant une caractéristique essentielle de l'invention, lesdites branches 5 et le culot 7 constituent une cage 1 creuse dont, en position « repos », comme représenté suivant les modes de réalisation des figures 1, 3A, 4A et 5A et des figures 9, 10 et 11, la forme générale extérieure est un cylindre ou un quasi cylindre dont la section transversale qui est aussi la directrice dudit cylindre, est circulaire ou quasi circulaire, et dont la génératrice qui s'appuie sur ladite directrice et qui engendre le cylindre ou quasi cylindre en tournant autour de son axe de symétrie XX', est soit une droite, soit un arc de cercle convexe et de grand rayon : on obtient ainsi, soit un cylindre régulier de révolution tel que représenté en traits pleins 20₁ sur la figure 3A, soit un pseudo-cylindre que l'on qualifie dans la présente invention d' « ovale » ou « ovoïde », de forme externe donc légèrement bombée, tel que représenté en traits pointillés et mixtes 20₂ sur la figure 3A ; une partie au moins du volume intérieur 9 de la cage 1 vers les extrémités distales des branches 5, est en forme de tronc de cône quasi de révolution dont la plus grande base est du côté dudit culot 7, lequel implant comprend au moins quatre branches 5 et au moins une cale 2 apte à passer dans ledit orifice 8 et par la grande base du tronc de cône dans ledit volume intérieur 9.

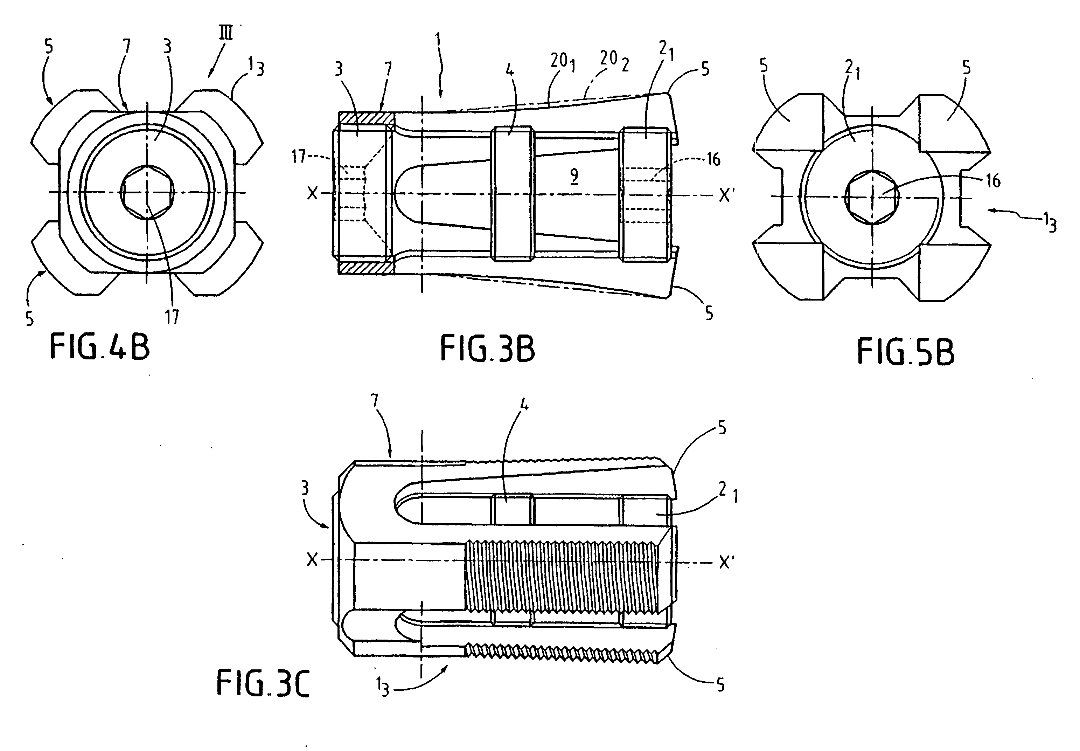

Suivant les figures 2, 3B, 4B, 5B et 3C, soit en position « active », ladite cale d'extrémité 2 écarte lesdites branches 5, ledit volume intérieur 9 tendant alors vers un cylindre de révolution et la forme extérieure de la cage 1 vers un quasi tronc de cône ; suivant la figure 3B par exemple, on a représenté en traits pleins 20₁ la forme légèrement concave obtenue à partir d'un cylindre de base régulier en position repos, et en traits pointillés mixtes 20₂, de forme plus droite obtenue à partir d'un cylindre ovoïde initial, tel que représenté à partir de la figure 3A ; une fois la cale 2 disposée à la position voulue, aucune pièce interne, ayant servi à la mise en place de l'implant et des cales, ne subsiste dans l'espace délimité par lesdites branches 5, la cale 2 et l'orifice 8.

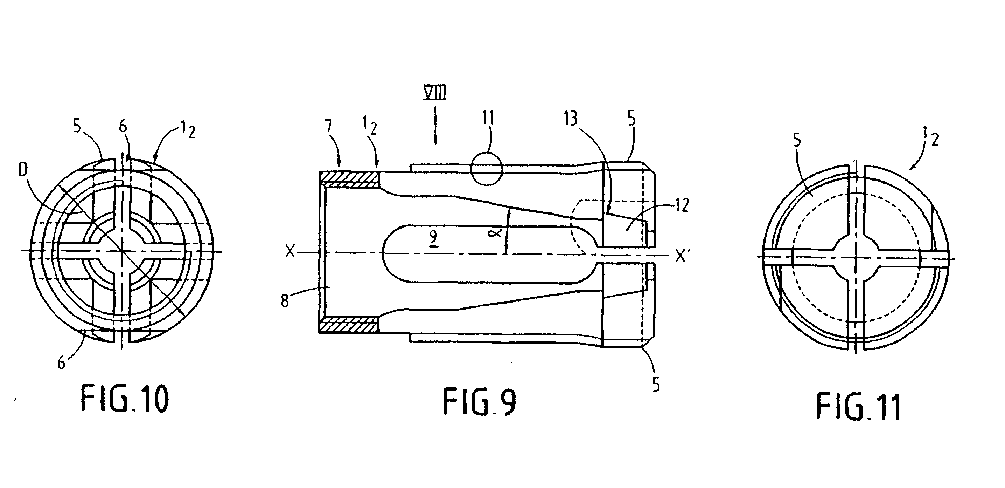

Quel que soit le mode de réalisation, au moins une partie de la surface externe desdites branches 5 est filetée suivant un profil de filet 11 à arêtes saillantes, telle que représentée en détail sur la figure 7 : en particulier, à titre d'exemple, pour une longueur L de cage de l'ordre de 20 à 25 millimètres, la longueur 1 de la partie filetée des branches 5 peut être de 13 à 16 millimètres avec un pas de filetage p de 1,5 à 2 millimètres, un diamètre externe D du cylindre de révolution de la cage de 9 à 16 millimètres et une hauteur de dent 11₁ de filetage de l'ordre de 0,7 à 0,9 millimètre pour un angle d'ouverture β entre chaque dent de 60° environ et un profil 11₂ intérieur de filetage d'un rayon de l'ordre de 0,4 millimètre ; ladite forme arrondie du profil réduisant au maximum les concentrations de contraintes et permettant ainsi de résister aux efforts importants et aux chocs.

Un tel filetage extérieur à arêtes saillantes permet ainsi une mise en place facilitée car non traumatisante puisqu'il n'y a pas de choc d'impact pour insérer ledit implant qui est ainsi vissé dans un trou de foret préalable grâce à tout outil compatible avec l'orifice 8 de l'implant et d'autre part, un tel filetage assure après mise en place, l'ancrage dans l'os, évitant toute migration ultérieure.

Le culot 7 peut comporter au moins deux méplats 6 parallèles ou légèrement inclinés l'un par rapport à l'autre pour mieux s'adapter au profil des vertèbres, disposés chacun entre deux branches 5 successives telles que représentées sur les modes de réalisation des figures 1, 2, 9, 10 et 11 ; également, le culot 7 peut comporter quatre méplats formant une section carrée ou pseudo-carrée, telle que représentée sur les modes de réalisation des figures 3, 4 et 5 ; en plus du culot, les branches 5 elles-mêmes, surtout si la section de l'implant est quasi circulaire, peuvent elles aussi comporter un méplat dans le prolongement d'au moins ceux du culot, comme représenté sur les exemples de réalisation des figures 3 à 5, ou ne pas comporter du tout de méplat, comme l'exemple des figures 1, 2 et 9 à 11 ; de tels méplats peuvent être remplacés ou tout au moins complétés par une section longitudinale de l'implant de forme légèrement ovale ou ovoïde telle qu'évoquée précédemment.

L'orifice 8 du culot 7 peut être fileté suivant un profil de filetage 15 tel que représenté à titre d'exemple sur la figure 6, suivant un pas de filet arrondi, aussi bien en sommet qu'en fond de gorge avec par exemple, pour les exemples dimensionnels donnés précédemment, une ouverture 8 de diamètre intérieur d de 7 à 10 millimètres, un pas p' de 1 à 1,5 millimètre, une hauteur de filetage de 0,6 millimètre environ et un angle γ entre les parois des gorges de filetage de l'ordre de 30°.

Un bouchon 3 de fermeture du volume intérieur 9 se visse alors dans ledit orifice 8, soit pour servir de point d'ancrage à une tige de mise en place de l'implant, soit après la mise en place de celui-ci et l'ouverture des branches par la cale d'extrémité 2 pour fermer le volume intérieur 9, afin d'une part de rigidifier la cage et d'autre part d'empêcher la matière osseuse que l'on peut implanter dans la cage, de ressortir par cette extrémité postérieure.

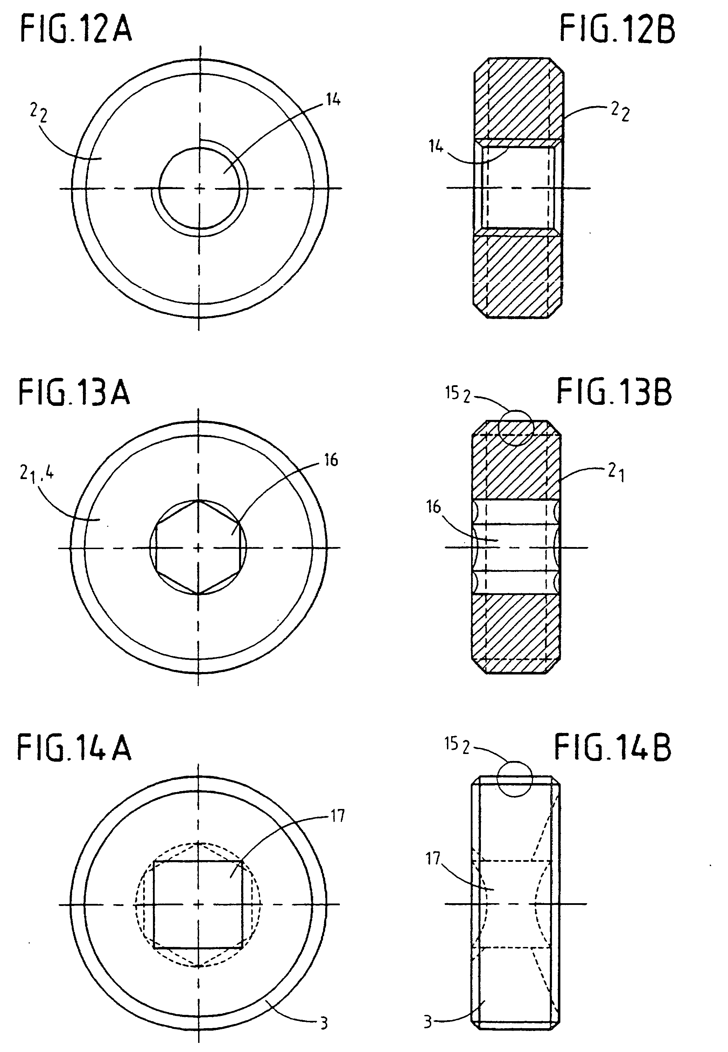

Un tel bouchon 3 est représenté vu de face sur la figure 14A et vu de profil sur la figure 14B, avec un filetage 15₂ du type de celui représenté sur la figure 6 et un orifice central 17 de forme polygonale, soit carrée, hexagonale, etc... ou remplacé par tout moyen pour y bloquer l'extrémité d'une tige ayant une extrémité compatible afin de pouvoir le visser ou le dévisser.

De plus, il peut être réalisé sur la face postérieure de l'implant et à la périphérie de l'orifice 8 du culot 7, des ergots ou des rainures permettant d'y immobiliser une partie de l'appareil de mise en place, dit ancillaire, autour de la tige permettant la manoeuvre de la cage 1, des cales et/ou du bouchon 3, afin de mieux figer le positionnement de l'implant durant sa mise en place et de faciliter le démontage de l'ancillaire sans faire bouger l'implant.

Dans les modes de réalisation représentés avec quatre branches 5, ladite cage 1 comporte quatre lumières 10 formant l'espace inter-branches tel que représenté sur la figure 8 suivant la vue 8 de la figure 9 par exemple : de telles lumières permettent d'une part une meilleure fusion du greffon osseux pouvant être logé dans le volume intérieur 9 par rapport aux disques intervertébraux adjacents, et d'autre part une meilleure déformation des branches 5 lors de la mise en place de l'implant, la section des branches dans cette partie là étant en effet plus faible qu'à leur extrémité ; de plus, de telles lumières peuvent être de forme oblongue avec l'extrémité, située vers celle des extrémités distales des branches 5, plus étroite que l'autre extrémité telle que représentée sur la figure 8, et se terminant même par une légère fente 10₁ entre les deux extrémités distales desdites branches adjacentes ; une telle forme, au niveau tout au moins de la fenêtre principale 10, permet de retrouver une rainure aux bords parallèles après expansion de la cage ; de plus, un tel choix de profil, plutôt qu'une rainure initiale constante telle que représentée sur la figure 3A, permet d'augmenter la surface d'appui entre la cale 2 et les extrémités distales des branches 5 de la cage, offrant alors une meilleure résistance.

Suivant le mode de réalisation des figures 1, 2, 9, 10 et 11, la surface interne des branches 5 délimitant le volume intérieur 9 de la cage 1 est lisse, lequel volume comporte alors à son extrémité distale un logement 12 axial apte à recevoir ladite cale 2₂ telle que représentée sur la figure 12, et à la maintenir par un épaulement 13 de diamètre plus large que celui du volume intérieur 9 de la cage en position « active », telle que représentée sur la figure 2.

Ladite cale 2₂ comporte un alésage 14 axial fileté, apte à recevoir une tige dont au moins l'extrémité est également filetée et compatible pour la mise en place de la cale par simple poussée et déplacement en translation, ladite tige de mise en place étant ensuite amovible.

Suivant les figures 1 et 2, afin d'éviter, sous de fortes sollicitations dues à la pression des vertèbres adjacentes, tout risque de déformation du corps de la cage 1 à son extrémité antérieure vers les extrémités distales des branches 5 qui pourraient en effet se rapprocher les unes des autres en glissant autour de ladite cale 2, celle-ci peut comporter au moins deux ailettes 18 ou d'autres moyens, symétriques par rapport à l'axe XX' de l'implant ; soit une telle cale est positionnée dans la cage 1 avant la mise en place de l'implant, en l'enfilant du côté des branches, soit le culot 7 comporte au moins deux rainures 19 compatibles, dans lesquelles peuvent se glisser lors de la mise en place de ladite cale 2, si on veut introduire celle-ci après la cage, les deux dites ailettes 18, celles-ci ayant une épaisseur e au plus égale à la distance et à l'écartement entre les extrémités des branches distales des deux branches 5 adjacentes.

D'autres systèmes peuvent compléter le dispositif pour que les branches ne se déforment pas après leur expansion, tel qu'un anneau extérieur logé dans une gorge à leurs extrémités et déformable par celles-ci.

Suivant les modes de réalisation représentés sur les figures 3, 4 et 5, la surface intérieure des branches 5 délimitant le volume intérieur 9 de la cage 1, est filetée suivant un pas équivalent à celui de l'orifice 8 du culot 7 tel que représenté sur la figure 6 par exemple, et ladite cale 2₁ l'est également de façon compatible, comme représenté sur les figures 13A et 13B.

Dans ce mode de réalisation avec filetage intérieur, ladite cage 1 peut également comporter au moins une autre cale 4 intermédiaire, filetée comme celle de l'extrémité 2₁ et qui peut être vissée derrière celle-ci afin d'une part de venir comprimer de la matière osseuse que l'on aurait insérée dans l'espace délimité entre ces deux cales, et d'autre part de rigidifier la partie centrale de la cage.

Lesdites cales d'extrémité 2₁ et intermédiaire 4 comportent un orifice 16 axial de forme polygonale, apte à recevoir une tige amovible ayant une extrémité de forme mâle compatible pour assurer leur vissage et leur mise en place.

La forme en tronc de cône du volume intérieur 9 de la cage 1 peut être d'un demi-angle de pente α compris entre 6 et 9 degrés par exemple.

## Revendications

1. implant intervertébral comprenant une cage 1 extensible comportant des branches (5) reliées à un culot (7) percé d'un orifice (8), et au moins une cale (2, 2₁,4) apte à écarter lesdites branches **caractérisé en ce que** lesdites branches et ledit culots constituent une cage creuse dont la forme générale extérieure, en position de repos, présente une forme générale extérieure est un quasi cylindre de section quasi circulaire et dont une partie au moins du volume intérieur (9) de la cage (1) vers les extrémités distales des branches (5) est en forme de tronc de cône quasi de révolution dont la plus grande base est du côté dudit culot (7), lequel implant comprend au moins 3 branches (5) et au moins une cale (2) compatible avec les dimensions de la grande base du tronc de cône dans ledit volume intérieur (9), et **en ce que** ladite cage étant apte à maintenir ladite cale (2) et aucune pièce interne de liaison ne subsistant entre ladite cale (2) et l'orifice (8) une fois ladite cale disposée à la position voulue, et **en ce que** l'espace délimité par lesdites branches, dite cale et dit orifice du culot étant apte à recevoir de la matière osseuse.

2. Implant selon la revendication 1, dans lequel lesdites branches délimitent un volume intérieur (9) qui comporte un logement (12) maintenant la cale (2) dans une position où la cale écarte les branches dans une position finale d'expansion de l'implant.

3. Implant suivant la revendication 1 ou 2, **caractérisé en ce que** la surface interne des branches (5) délimitant le volume intérieur (9) de la cage (1) est lisse, la cale étant déplaçable en translation dans le volume intérieur par simple poussée.

4. Implant selon la revendication 2 ou 3, dans lequel une partie au moins du volume intérieur (9) est quasi de révolution et évasé du côté dudit culot.

5. Implant selon l'une quelconque des revendications 1 à 4, qui comporte au moins trois branches.

6. Implant selon l'une quelconque des revendications 1 à 5, qui comporte au moins quatre branches.

7. Implant selon l'une quelconque des revendications 1 à 6, qui comporte des lumières (10) inter-branches terminées par une fente (101) entre les extrémités de branches adjacentes opposées au culot.

8. Implant selon l'une quelconque des revendications 1 à 7, dans lequel les branches et le culot constituent une cage creuse dont la forme générale extérieure au repos est un cylindre ou un quasi cylindre.

9. Implant selon l'une quelconque des revendications 1 à 7, dans lequel les branches et le culot constituent une cage creuse dont la forme générale extérieure au repos est ovoïde.

10. Implant selon l'une quelconque des revendications 1 à 9, dans lequel les branches et le culot constituent une cage creuse dont la forme générale extérieure au repos est de révolution.

11. Implant suivant l'une quelconque des revendications 1 à 10, **caractérisé en ce que** au moins une partie de la surface externe desdites branches (5) est filetée.

12. Implant suivant l'une quelconque des revendications 1 à 11, **caractérisé en ce que** le culot (7) comporte au moins deux méplats (6) inclinés disposés chacun entre deux branches (5) successives.

13. Implant suivant l'une quelconque des revendications 1 à 12, **caractérisé en ce que** l'orifice (8) du culot (7) est apte à recevoir un bouchon (3) de fermeture du volume intérieur (9) de la cage (1).

14. Implant suivant l'une quelconque des revendications 1 à 13, **caractérisé en ce que** ladite cale (2, 4) comporte un orifice (16) axial, apte à recevoir l'extrémité d'une tige amovible de mise en place.

15. Implant suivant l'une quelconque des revendications 1 à 14, **caractérisé en ce que** ladite cale (2) comporte au moins deux ailettes (18) symétriques par rapport à l'axe de l'implant.

16. Implant suivant la revendication 15, dans lequel les ailettes ont une épaisseur e au plus égale à la distance de l'ouverture voulue entre les extrémités distales de deux branches (5) adjacentes.

17. Implant suivant l'une quelconque des revendications 1, 2 ou 4 à 16, **caractérisé en ce que** la surface interne des branches (5) est filetée, et ladite cale (2) également, d'une manière compatible.

18. Implant suivant la revendication 17, **caractérisé en ce qu'**il comporte au moins une deuxième cale (4).

19. Implant suivant l'une quelconque des revendications 1 à 18, **caractérisé en ce que** ladite cale (2) comporte un alésage (14) axial, fileté, apte à recevoir une tige amovible de mise en place dont l'extrémité au moins est également filetée et compatible.

20. Implant selon l'une quelconque des revendications 1 à 19, dans lequel lesdites branches (5) et le culot (7) constituent une cage (1) creuse dont, en position de repos, la forme générale extérieure est un quasi cylindre de section quasi circulaire et une partie au moins du volume intérieur (9) de la cage (1) vers les extrémités distales des branches (5) est quasi de révolution et évasée du côté dudit culot (7), lequel implant comprend au moins trois branches (5), ladite cale (2) étant compatible avec les dimensions dudit volume intérieur (9), et telle qu'en position active où ladite cale (2) écarte lesdites branches (5), ledit volume intérieur (9) tend vers un cylindre de révolution, et la forme extérieure de la cage (1) vers un quasi tronc de cône, et aucune pièce interne ne subsiste dans l'espace délimité par les branches, la cale et l'orifice.

21. Implant selon l'une quelconque des revendications 1 à 20, dans lequel le déplacement de la cale provoque une expansion bilatérale de l'implant en position active de celui-ci.

22. Implant selon l'une quelconque des revendications 1 à 20, dans lequel le déplacement de la cale provoque une augmentation du diamètre de l'extrémité distale de l'implant en position active de celui-ci.

## Patentansprüche

1. Intervertebrales Implantat, das einen expandierbaren Käfig 1 umfasst, der Arme (5) umfasst, die mit einem Sockel (7) verbunden sind, in den eine Öffnung (8) gebohrt ist, und mindestens einen Keil (2, 2₁, 4), der die Arme beabstanden kann, **dadurch gekennzeichnet, dass** die Arme und der Sockel einen Hohlkäfig bilden, dessen allgemeine äußere Form in Ruhestellung eine allgemeine äußere fast zylindrische Form mit fast kreisförmigem Querschnitt aufweist, und von dem zumindest ein Teil des Innenvolumens (9) des Käfigs (1) zu den distalen Enden der Arme (5) fast die Form eines Rotationskegelstumpfs hat, dessen größere Basis auf der Seite des Sockels (7) liegt, wobei das Implantat mindestens drei Arme (5) und mindestens einen Keil (2) umfasst, der mit den Maßen der großen Basis des Kegelstumpfs im Innenvolumen (9) vereinbar ist, und dadurch, dass der Käfig den Keil (2) halten kann und kein inneres Verbindungsteil zwischen dem Keil (2) und der Öffnung (8) mehr besteht, sobald der Keil in der gewünschten Stellung angeordnet ist, und dadurch, dass der von der Armen, dem Keil und der Öffnung des Sockels abgegrenzte Raum Knochenmaterial aufnehmen kann.

2. Implantat nach Anspruch 1, bei dem die Arme ein Innenvolumen (9) abgrenzen, das eine Aufnahme (12) umfasst, die den Keil (2) in einer Stellung hält, in der der Keil die Arme in einer Endausdehnungsstellung des Implantats beabstandet.

3. Implantat nach Anspruch 1 oder 2, **dadurch gekennzeichnet, dass** die Innenfläche der Arme (5), die das Innenvolumen (9) des Käfigs (1) begrenzen, glatt ist, wobei der Keil translatorisch in dem Innenvolumen durch einfachen Druck verschieblich ist.

4. Implantat nach Anspruch 2 oder 3, bei dem zumindest ein Teil des Innenvolumens (9) fast rotationsförmig und auf der Seite des Sockels aufgedehnt ist.

5. Implantat nach einem der Ansprüche 1 bis 4, das mindestens drei Arme umfasst.

6. Implantat nach einem der Ansprüche 1 bis 5, das mindestens vier Arme umfasst.

7. Implantat nach einem der Ansprüche 1 bis 6, das Langlöcher (10) zwischen den Armen umfasst, die in einem Schlitz (101) zwischen den Enden nebeneinander liegender, dem Sockel gegenüber liegender Arme enden.

8. Implantat nach einem der Ansprüche 1 bis 7, bei dem die Arme und der Sockel einen Hohlkäfig bilden, dessen allgemeine äußere Form in der Ruhestellung ein Zylinder oder fast ein Zylinder ist.

9. Implantat nach einem der Ansprüche 1 bis 7, bei dem die Arme und der Sockel einen Hohlkäfig bilden, dessen allgemeine äußere Form in der Ruhestellung eiförmig ist.

10. Implantat nach einem der Ansprüche 1 bis 9, bei dem die Arme und der Sockel einen Hohlkäfig bilden, dessen allgemeine äußere Form in der Ruhestellung eine Rotationsform ist.

11. Implantat nach einem der Ansprüche 1 bis 10, **dadurch gekennzeichnet, dass** mindestens ein Teil der äußeren Oberfläche der Arme (5) einen Gewindeschnitt trägt.

12. Implantat nach einem der Ansprüche 1 bis 11, **dadurch gekennzeichnet, dass** der Sockel (7) mindestens zwei schräge Flachteile (6) umfasst, die jeweils zwischen zwei aufeinander folgenden Armen (5) angeordnet sind.

13. Implantat nach einem der Ansprüche 1 bis 12, **dadurch gekennzeichnet, dass** die Öffnung (8) des Sockels (7) einen Stopfen (3) zum Verschließen des Innenvolumens (9) des Käfigs (1) aufnehmen kann.

14. Implantat nach einem der Ansprüche 1 bis 13, **dadurch gekennzeichnet, dass** der Keil (2, 4) eine axiale Öffnung (16) umfasst, die das Ende eines abnehmbaren Schafts zum Anbringen aufnehmen kann.

15. Implantat nach einem der Ansprüche 1 bis 14, **dadurch gekennzeichnet, dass** der Keil (2) mindestens zwei Flügel (18) umfasst, die in Bezug auf die Achse des Implantats symmetrisch sind.

16. Implantat nach Anspruch 15, bei dem die Flügel eine Stärke (e)haben, die maximal gleich der Entfernung der gewünschten Öffnung zwischen den distalen Enden zweier nebeneinander liegender Armen (5) ist.

17. Implantat nach einem der Ansprüche 1, 2 oder 4 bis 16, **dadurch gekennzeichnet, dass** die Innenfläche der Arme (5) und in kompatibler Weise auch der Keil (2) einen Gewindeschnitt tragen.

18. Implantat nach Anspruch 17, **dadurch gekennzeichnet, dass** es mindestens einen zweiten Keil (4) umfasst.

19. Implantat nach einem der Ansprüche 1 bis 18, **dadurch gekennzeichnet, dass** der Keil (2) eine axiale Gewindebohrung (14) umfasst, die einen abnehmbaren Schaft zum Anbringen aufnehmen kann, von dem mindestens das Ende ebenfalls einen Gewindeschnitt trägt und kompatibel ist.

20. Implantat nach einem der Ansprüche 1 bis 19, bei dem die Arme (5) und der Sockel (7) einen Hohlkäfig (1) bilden, dessen allgemeine äußere Form in der Ruhestellung fast zylindrisch mit fast kreisförmigem Querschnitt ist und mindestens ein Teil des Innenvolumens (9) des Käfigs (1) zu den distalen Enden der Arme (5) hin fast Rotationsform hat und auf der Seite des Sockels (7) aufgedehnt ist, wobei das Implantat mindestens drei Arme (5) umfasst, wobei der Keil (2) mit den Maßen des Innenvolumens (9) kompatibel ist und so, dass das Innenvolumen (9) in der aktiven Stellung, in der der Keil (2) die Arme (5) beabstandet, zu einem Rotationszylinder tendiert und die äußere Form des Käfigs (1) zu einem Quasi-Kegelstumpf, und wobei in dem von den Armen, dem Keil und der Öffnung abgegrenzten Raum kein anderes Innenteil verbleibt.

21. Implantat nach einem der Ansprüche 1 bis 20, bei dem das Verschieben des Keils in seine aktive Position eine beidseitige Dehnung des Implantats bewirkt.

22. Implantat nach einem der Ansprüche 1 bis 20, bei dem das Verschieben des Keils in seine aktive Position eine Steigerung des Durchmessers des distalen Endes des Implantats bewirkt.

## Claims

1. An intervertebral implant comprising an expandable cage (1) including branches (5) each connected to a seat (7) pierced by an orifice (8), and at least one spacer (2, 2₁, 4) adapted to splay said branches apart, said implant being **characterised in that** said branches and said seat constitute a hollow cage which, in a rest position, has an outside general shape which is a quasi-cylinder of quasi-circular section, and a portion at least of the inside volume (9) of the cage (1) towards the distal ends of said branches (5) is in the form of a quasi-circular truncated cone whose large base is towards said seat (7), which implant has at least three branches (5) and, inside said inside volume (9), at least one spacer (2) compatible with the dimensions of the large base of the truncated cone, and **in that** said cage being adapted to hold said spacer (2) and no internal link part remaining between said spacer (2) and the orifice (8) once said spacer has been placed in the desired position, and **in that** the space defined by said branches, said spacer and said orifice of the seat being adapted to receive bone matter.

2. An implant according to claim 1, in which said branches define an inside volume (9) which has a housing (12) for holding the spacer (2) in a position in which the spacer splays the branches apart in a final expansion position of the implant.

3. An implant according to claim 1 or 2, **characterised in that** the inside surfaces of the branches (5) defining the inside volume (9) of the cage (1) are smooth, with the spacer being moved in translation in the inside volume by mere thrust.

4. An implant according to claim 2 or 3, in which at least a portion of the inside volume (9) is quasi-circular and flares away from the seat.

5. An implant according to any one of claims 1 to 4, which includes at least three branches.

6. An implant according to any one of claims 1 to 5, which includes at least four branches.

7. An implant according to any one of claims 1 to 6, which includes inter-branch slots (10) terminating in a narrow slit (101) between the ends of adjacent branches opposite the seat.

8. An implant according to any one of claims 1 to 7, in which the branches and the seat constitute a hollow cage which, in a rest position, has an outside general shape which is a cylinder or a quasi-cylinder.

9. An implant according to any one of claims 1 to 7, in which the branches and the seat constitute a hollow cage which, in a rest position, has an outside general shape which is ovoid.

10. An implant according to any one of claims 1 to 9, in which the branches and the seat constitute a hollow cage which, in a rest position, has an outside general shape which is circular.

11. An implant according to any one of claims 1 to 10, **characterised in that** at least a portion of the outside surfaces of said branches (5) is threaded.

12. An implant according to any one of claims 1 to 11, **characterised in that** the seat (7) has at least two flats (6) each disposed between two successive branches (5).

13. An implant according to any one of claims 1 to 12, **characterised in that** the orifice (8) of the seat (7) is suitable for receiving a plug (3) for closing the inside volume (9) of the cage (1).

14. An implant according to any one of claims 1 to 13, **characterised in that** said spacer (2, 4) includes an axial orifice (16) suitable for receiving the end of an installation rod that is removable.

15. An implant according to any one of claims 1 to 14, **characterised in that** said spacer (2) includes at least two splines (18) disposed symmetrically about the axis of the implant.

16. An implant according to claim 15, in which the width e of the splines is no greater than the desired gap distance between the distal ends of two adjacent branches (5).

17. An implant according to any one of claims 1, 2 or 4 to 16, **characterised in that** the inside surfaces of the branches (5) are threaded and said spacer (2) is also threaded, in compatible manner.

18. An implant according to claim 17, **characterised in that** it has at least one second spacer (4).

19. An implant according to any one of claims 1 to 18, **characterised in that** said spacer (2) includes a threaded axial bore (14) suitable for receiving an installation rod that is removable, at least the end of the rod being also threaded and compatible.

20. An implant according to any one of claims 1 to 19, in which said branches (5) and the seat (7) constitute a hollow cage (1) which, in a rest position, has an outside general shape which is a quasi-cylinder of quasi-circular section, and a portion at least of the inside volume (9) of the cage (1) towards the distal ends of said branches (5) is quasi-circular and flares away from said seat (7), which implant has at least three branches (5), said spacer (2) being compatible with the dimensions of said inside volume (9), and such that in an "active" position in which said spacer (2) splays said branches (5) apart, said inside volume (9) tends towards a circular cylinder while the outside shape of the cage (1) tends towards an approximate truncated cone, and no internal part remains in the space defined by the branches, the spacer, and the orifice.

21. An implant according to any one of claims 1 to 20, in which moving the spacer makes it possible to obtain bilateral expansion of the implant in the active position thereof.

22. An implant according to any one of claims 1 to 20, in which moving the spacer makes it possible to increase the diameter of the distal end of the implant in the active position thereof.
